(19) Europäisches Patentamt
European Patent Office
Office européen des brevets

(11) **EP 3 355 060 A1**

(12) **EUROPEAN PATENT APPLICATION**

(43) Date of publication:
**01.08.2018 Bulletin 2018/31**

(51) Int Cl.:
***G01N 33/72*** *(2006.01)*

(21) Application number: **17153796.2**

(22) Date of filing: **30.01.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
- **Deutsches Krebsforschungszentrum, Stiftung des öffentlichen Rechts**
  **69120 Heidelberg (DE)**
- **Albert-Ludwigs-Universität Freiburg**
  **79085 Freiburg (DE)**

(72) Inventors:
- **Rodriguez Gonzalez, Agustin**
  **69126 Heidelberg (DE)**
- **Schilling, Marcel**
  **69121 Heidelberg (DE)**
- **Klingmüller, Ursula**
  **69120 Heidelberg (DE)**
- **Steiert, Bernhard**
  **79108 Freiburg (DE)**
- **Timmer, Jens**
  **79102 Freiburg (DE)**

(74) Representative: **Krauss, Jan**
**Boehmert & Boehmert**
**Anwaltspartnerschaft mbB**
**Pettenkoferstrasse 22**
**80336 München (DE)**

(54) **PREDICTION-METHOD OF MORTALITY DUE TO TREATMENT WITH ERYTHROPOIESIS STIMULATING AGENTS**

(57) The present invention pertains to the diagnosis of a high risk of mortality or other adverse events in a patient suffering from anemia, for example anemia caused by chemotherapy, cancer or chronic inflammation. The invention provides means to diagnose a patient who receives Erythropoiesis Stimulating Agents (ESA) to suffer from an adverse event if the treatment with the ESA is continued. Based on the herein disclosed methods, the clinician will be able diagnose the prevalence of a fatal event and adjust the treatment of the anemia in the patient accordingly.

**EP 3 355 060 A1**

**Description**

FIELD OF THE INVENTION

The present invention pertains to the diagnosis of a high risk of mortality or other adverse events in a patient suffering from anemia, for example anemia caused by chemotherapy, cancer or chronic inflammation. The invention provides means to diagnose a patient who receives Erythropoiesis Stimulating Agents (ESA) to suffer from an adverse event if the treatment with the ESA is continued. Based on the herein disclosed methods, the clinician will be able diagnose the prevalence of a fatal event and adjust the treatment of the anemia in the patient accordingly.

DESCRIPTION

Many cytokines act systemically, bind to cell surface receptors on specific target cells and trigger their survival, proliferation and differentiation. Thereby highly-specialized cells are produced that fulfil essential functions in an organism. Hence, alterations in cellular responses may have major consequences at the body scale. Conversely, multiple cytokines or their derivatives are exploited as therapeutic agents and are systemically applied to elicit cellular responses and alleviate pathological conditions. Several non-linear reactions contribute to this intricate circuit and determine the outcome. Therefore, a rational approach for optimized treatment design is required, which necessitates detailed insights into molecular mechanisms and the development of a mathematical modelling concept that spans from the cellular to the body scale.

A therapeutically relevant cytokine is the hormone erythropoietin (Epo). The dynamic interactions of Epo with its cognate receptor, the erythropoietin receptor (EpoR), determine proliferation of erythroid progenitor cells at the colony forming unit erythroid (CFU-E) stage and their differentiation to short-lived mature erythrocytes that contain haemoglobin (Hb) and secure oxygen supply in the body Low-levels of erythrocytes which correspond to reduced Hb values are characteristic for anaemia that is for example frequently observed in lung cancer patients, reaching up to 90% at the advanced stages of the disease. Anaemia reduces the quality of life, increases mortality risk and diminishes chemotherapeutic effects. For anaemia treatment, erythropoiesis stimulating agents (ESAs) such as recombinantly produced Epo or Epo-derivatives are widely used. However, in the context of cancer-associated anaemia, ESA treatment is controversially discussed because clinical trials were terminated due to adverse effects and the EpoR was reported to be present on tumour cells. Apparently, EpoR levels on carcinoma cell lines are much lower compared to the expression on hCFU-E and their accurate detection remains challenging.

30-50% of lung cancer patients do not respond to ESA treatment. Linear or logistic regression models were developed to predict patient responses from clinical markers. However, none of the tested baseline parameters or their combination showed sufficient sensitivity for individualized prediction of the response. Similar attempts were performed to identify parameters with predictive values for risk assessment of thromboembolic events and mortality. Studies conducted in cancer and chronic kidney disease described a general correlation of hyporesponsiveness to ESA treatment with high ESA doses and mortality. However, due to the lack of patient-specific parameters that facilitate the individualized prediction of responses to ESAs, it was so far not possible to perform risk stratification of patients.

Based on data from clinical trials, mathematical models were developed that described the averaged pharmacokinetic (PK) and pharmacodynamic (PD) responses to ESA treatment at the body scale. However, none of these mathematical models contained biochemical reactions at the cellular scale. The inventorsrecently reported a quantitative dynamic pathway model, that describes the dynamic interaction of Epo with the murine EpoR (mEpoR), and thereby uncovered that rapid receptor turnover enables the system to respond to a broad range of ligand doses. Ligand binding to the receptor elicits the activation of signalling pathways including the JAK2-STAT5 signalling cascade. By dynamic pathway modelling the inventors showed that the extent of Epo-induced phosphorylation of STAT5 relates linearly to cell survival of CFU-E cells and that Epo stimulation enhances survival of the non-small cell lung cancer (NSCLC) cell line H838 upon treatment with the chemotherapeutic agent cisplatin. The cellular scale mathematical models provide mechanistic insights and facilitate quantitative predictions and therefore might provide essential modules for the development of predictive multiscale models for patient stratification, risk prediction and therapy optimization. Lung carcinoma is the most frequent cause of death in cancer with 1.59 million of deaths in 2012, of which 80% were diagnosed as Non-Small Cell Lung Carcinoma (NSCLC). Most of the patients are diagnosed in a stage IIIB or IV and treated with a combination of platinum compounds and taxanes, gemcitabine or vinorelbine as a first line of treatment. In lung carcinoma there is a high prevalence of anemia ([Hb]≤11g/dL), ranging from 50% to 70%, although in advanced stages it could reach up to 90%. The anemic grade depends on the therapy, tumor stage and duration of the disease. Cancer related anemia reduces the quality of life ( Cella et al, 2004) and it is considered a risk factor for mortality in cancer patients (Caro 2001). Furthermore, it has been reported that anemia affects the outcome of the anticancer therapy, diminishing the chemotherapy response in NSCLC patients (Albain 1991, MacRae 2002 and Robnett 2002).

The etiology of anemia in cancer is complex due to the multifactorial causes such as deficiencies in vitamin B12 and folic acid, bleeding, haemolysis, inflammatory cytokines secreted in the tumor context and reduction in the iron uptake (Weiss and Goodnough N.Engl. J Med 2005) are some of the causal origins of cancer related anemia. In adition, platinum-based chemotherapy inhibits the renal production of Epo and exerts myelosuppresion what increases the anemia (Groopman1999, Kosmidis 2005, Ludwig2004).

WO 2015/193462 describes mathematical models for the prediction of ESA concentrations for use in the treatment of anemia. The present invention is an additional development based on the technical teaching of WO 2015/193462. Therefore, WO 2015/193462 is incorporated herein by reference in its entirety.

In view of the existing problems of ESA treatment of anemia caused by cancer, chemotherapy, chronic inflammation or other primary disorders, it was an object of the present invention to provide a diagnostic approach to identify patients having an increased risk of mortality or other adverse events (such as thrombovascular events) upon ESA treatment.

In one aspect the above problem is solved by a method for stratifying an anemia patient who receives treatment with an erythropoiesis Stimulating Agents (ESA), wherein the patient is stratified into a high risk or low risk group of experiencing a fatal and/or adverse outcome upon continued treatment with the ESA, the method comprising the steps of:

(a) Providing patient samples of the patient from at least two time points during the initial treatment of anemia with the ESA in said patient,
(b) Determining from said samples the individual hemoglobin (Hb) degradation rate [Hb degr] and number of ESA binding sites [EpoR] for said patient,
(c) Determining from (i) the individual Hb degradation rate, (ii) the number of ESA binding sites, and (iii) the last ESA dose administered, or ESA dose planned/calculated to be administered, to said patient [ESA], an accumulated risk factor [aRF], and
(d) Stratifying the patient into a high risk or low risk group of experiencing an adverse event upon continued treatment with the ESA according to the [aRF].

In preferred embodiments the adverse event is selected from a fatal outcome such as the death of the patient, preferably death of the patient caused by ESA treatment. In other embodiments the adverse event is selected from thrombovascular events.

The method is preferably performed *in-vitro.*

The [aRF] is determined by a linear combination of (i) the individual Hb degradation rate, (ii) the number of ESA binding sites, and (iii) the last ESA dose administered, or ESA dose planned/calculated to be administered. Preferably, according to the following equation A:

$$\textbf{Equation A:}\quad [aRF]=B0+B1*[EpoR]+B2*[Hb\ degr]+B3*[ESA]$$

In preferred aspects the factors of the above equation A are B0=2.3518, B1=-2.5840, B2=-0.3957, and B3=-0.1374. Preferably, the factors may vary upon situation, but not more than +/- 20%, 1%, 10%, and preferably not more than 5% from the above indicated value. These are particularly useful in the event the patient is treated with CERA and suffers from NSCLC.

In preferred embodiments of the invention, a patient is at a high risk to experience an adverse event if [aRF] is 0.1 or higher, preferably 0.15 or higher, or 0.17 or higher, and most preferably wherein [aRF] is > about 0.18.

In context of the present invention the number of ESA binding sites [EpoR] for the patient is determined by assessing the clearance of the administered ESA in the serum of said patient over time, and calculating from the clearance of said ESA using a non-linear dynamic pharmacokinetic (PK) ESA-EPO-R pathway model the amount of ESA binding sites in said patient [EpoR]. The models are described herein below.

Preferred are methods wherein the individual hemoglobin (Hb) degradation rate [Hb degr] is determined by calculating from the hemoglobin concentration of the patient from at least two separate time points the patient's individual hemoglobin degradation rate (degradation of hemoglobin per time).

In preferred embodiments said non-linear dynamic pharmacokinetic (PK) ESA-EPO-R pathway model is based on a system of the ordinary differential equations (ODE) as described herein below

In context of the herein described invention the hemoglobin concentration of the patient (or subject, terms which are used herein as synonyms) is preferably determined through blood samples taken from the patient. Methods for calculating the haemoglobin concentrations are well known in the art. Alternatively, since most anemia patients have a treatment history where haemoglobin concentrations were determined at multiple time points, the patients hemoglobin degradation rate may be calculated from these values taken from the individual patient's medical file.

The hemoglobin degradation rate may either be determined by measuring hemoglobin concentrations in the patient at several time points, for example in an ESA naive or ESA receiving patient, or using the patient's previous treatment history. In accordance with the herein described mathematical model the specific characteristics of the ESA to be used in therapy, for example CERA , but other ESA are also included in the invention, are used for determining the ESA dosage.

Based in the initial experiments *in vitro* (ESA depletion experiments) as described in the example section, the mathematical model as disclosed describes the binding properties of each ESA: the association rate "$k_{on}$" and the dissociation rate "$k_{off}$" (the dissociation constant "$K_D$" is defined as koff/kon). Based in the binding properties of each ESA, the herein disclosed model can calculate the integral occupancy of the EpoR on human CFU-E for 60 minutes. The $EC_{50}$ (ESA concentration required to obtain half-maximum EpoR occupancy) is calculated for each ESA and this correlates with the ESA activity in hCFU-E. In the integrative non-linear dynamic pharmacokinetic (PK) hemoglobin (Hb) ESA-EPO-R pathway model, the integral occupancy of the ESA-EpoR is linked to Hb production. The amount of ESA-EpoR is, among all the other parameters, depending on the $k_{on}$ and the $k_{off}$ rate of the specific ESA. Based on the ESA depletion experiments, the mathematical model calculates $k_{on}$ and $k_{off}$ for each ESA. This data can be used (i) to calculate $EC_{50}$ values for each ESA and (ii) calculate Hb values based on ESA injections. Thereby, the using the non-linear dynamic pharmacokinetic (PK) hemoglobin (Hb) ESA-EPO-R pathway model of the invention, the ESA dosage for achieving a production of hemoglobin in the anemia patient that is sufficient to alleviate the anemia can be calculated.

The term "anemia" in context of the herein described invention shall refer to a condition wherein the red blood cells are reduced. Anemia is typically diagnosed on a complete blood count. Apart from reporting the number of red blood cells and the hemoglobin level, the automatic counters also measure the size of the red blood cells by flow cytometry, which is an important tool in distinguishing between the causes of anemia. Examination of a stained blood smear using a microscope can also be helpful, and it is sometimes a necessity in regions of the world where automated analysis is less accessible. In modern counters, four parameters (RBC count, hemoglobin concentration, MCV and RDW) are measured, allowing others (hematocrit, MCH and MCHC) to be calculated, and compared to values adjusted for age and sex. Some counters estimate hematocrit from direct measurements. In the context of the present invention anemia is present if an individual has a hemoglobin (Hb) concentration of less than 14 g/dL, more preferably of less than 12 g/dL, most preferably of less than 11 g/dL.

In certain embodiments of the invention the anemia to be treated in accordance with the described methods is an anemia that has developed according to any possible cause or disease. This includes all types of cancer, all inflammation-associated anemia (chronic infection disease, autoimmune or rheumatologic disorders and any other illnesses or treatments that results in anemia based on reduced endogenous Epo production, inefficient eryhtropoiesis or increased destruction of red blood cells). Furthermore and particularly preferred, is that the anemia is caused by chemotherapy, chronic kidney disease (CKD), myelodysplastic syndrome (MDS), or is anemia associated to myelofibrosis, anemia in context of HIV, aplastic anemias, anemia in premature infants, non-severe aplastic anemia, anemia in beta thalassemia, anemia in sickle cell disease and ESA erythropoiesis stimulation after allogeneic hematopoietic stem cell transplantation.

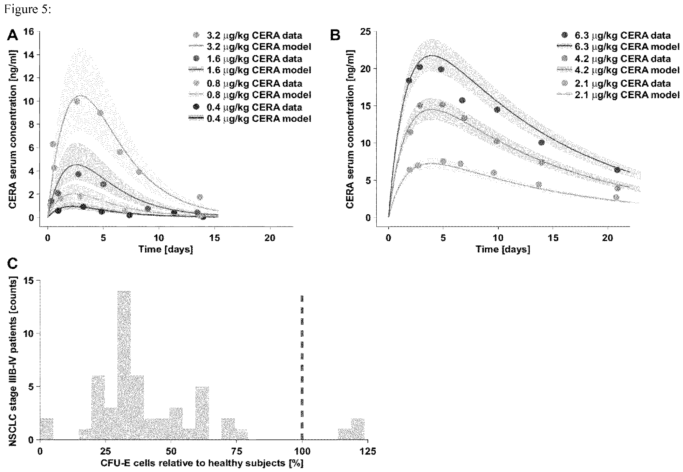

The inventors of the present invention previously discovered that a mathematical model describing the EPO-EPO-R signaling pathway in a cell can be adapted to predict the behavior of not only ESAs in a cell, but also of the dynamics of ESAs administered to a patient. Initially the model is able to describe at cellular level the activity of the different ESAs based in the affinity of each ESA (time of EpoR occupancy). This activity corresponds to the EPO-R activation by ESA binding to the EPO receptor. This activation of the EPO-R will induce the proliferation and maturation of the erythropogenitors, the main cellular population on the body that express EpoR into erythrocytes. For the present invention the initial core model that describes the EpoR activation at cellular level by ESA was extended in order to be used in a physiological situation in an organism, in particular a human patient. Clearance of an administered ESA in the blood compartment, transport of an subcutaneous administered ESA into the blood compartment and saturable clearance of the ESA in the interstitial compartment were added to the initial model. This extended version of the initial ESA-EPO-R model was surprisingly able to describe the published pharmacokinetic (PK) and pharmacodynamics (PD) experimental data of each ESA as shown in the examples. The inventors could characterize induced anemia by cancer and chemotherapy in individual patients at colony forming unit of erythroids (CFU-E), the progenitors of the erythroids. It was observed that patients in the same cancer type and disease stage (figure 5c) show different numbers of CFU-E. This explains the different ESA treatment outcomes observed in patients - 40% of the NSCLC patients do not respond to ESA treatment in the current approved posology (protocol to treat anemic patients with cancer). Lower levels of CFU-E means lower levels of response to ESA treatments and it correlated with the individual outcomes at hemoglobin levels (Hb). Now, in context of the present invention, it was surprising that using the number of EPO binding sites and the hemoglobin degradation rate of a patient in combination with a planned ESA treatment dosage allows to adequately predicting whether the patient will suffer from a fatal event due to ESA treatment. Using the methods of the invention allows a clinician to decide whether or not to continue an ESA treatment in an anemic patient, or whether it is better to resort to blood transfusions. The basic calculations of the accumulated risk factor are provided herein below.

In the context of the invention which is described in the following, the mathematical models are all based on the basic findings as published and publically accessible in the publication Becker V et al., Science. 2010 Jun 11;328(5984):1404-8 and the publication WO 2015/193462 . These references are incorporated in their entirety, for the purpose of understanding the application of the methods of the present invention. The models used in context of the present invention were adjusted to answer the respective questions of the herein disclosed invention. In this respect the term "non-linear dynamic EPO-EPO-R pathway model" shall refer to the model as published by the above Becker V et al. 2010 reference. The term "non-linear dynamic ESA-EPO-R pathway model" shall refer to the version of the non-linear dynamic EPO-EPO-R pathway model in WO 2015/193462, which describes the binding/dissociation dynamics of ESAs to the EPO-R on a cellular level. The term "non-linear dynamic pharmacokinetic ESA-EPO-R pathway model" shall refer to the non-linear dynamic ESA-EPO-R pathway model in WO 2015/193462 which is adjusted to the situation in an organism, in particular a human patient. The basic rationales for the models disclosed herein are provided in the Materials and Methods section of the present application.

Thus it is a preferred embodiment that the non-linear dynamic pharmacokinetic (PK) ESA-EPO-R pathway model considers clearance of the administered ESA in the blood compartment, transport of the administered ESA from the interstitial compartment into the blood compartment, and clearance of the ESA in the interstitial compartment.

The basic application of the mathematical methods as required by the herein described inventive methods is standard to the person of skill in the field of systems biology. Using the information as provided by the present patent application, the person of skill in view also of the Becker V et al. 2010 publication can perform the necessary steps to work the invention.

For the present disclosure the following variables, constants and acronyms are used:

**Table 1: Acronyms**

| | |
|---|---|
| CFU-E | Colony forming unit-erythroid |
| NSCLC | Non-small cell lung carcinoma |
| Hb | Hemoglobin |
| RBC | Red blood cells |
| Epo | Erythropoietin |
| EpoR | Erythropoietin receptor |
| PK | Pharmacokinetics |
| PD | Pharmacodynamics |
| MEPC | Minimal Personal Effective ESA Concentration |
| CKD | Chronic kidney disease |
| MDS | Myelodysplastic syndrome |
| NESP | Novel erythropoiesis stimulating protein |
| CERA | Continuous erythropoietin receptor activator |
| STAT5 | Signal transducer and activator of transcription 5 |
| EC50 | Half-maximal effective concentrations |
| ODE | Ordinary differential equation |
| U | Units |

**Table 2: Variables**

| | |
|---|---|
| ESA | Erythropoiesis-stimulating agent in medium / blood |
| Epo | Erythropoietin |
| EpoR | Erythropoietin receptor |
| ESAEpoR | Complex of ESA bound to EpoR on the cell surface |
| $ESAEpoR_i$ | Internalized complex of ESA bound to EpoR |
| $dESA_i$ | Intracellular degraded ESA |
| $dESA_e$ | Extracelullar degraded ESA |

(continued)

| ESA$_{SC}$ | ESA in the subcutaneous compartment |
|---|---|
| Hb | Hemoglobin in blood |

**Table 3: Kinetic constants**

| $k_{sc\_clear}$ | ESA clearance constant in the subcutaneous compartment |
|---|---|
| $k_{sc\_clear\_sat}$ | Saturation of ESA clearance in subcutaneous compartment |
| $k_{sc\_out}$ | ESA transportation constant to the blood compartment |
| $k_{clear}$ | ESA clearance constant in the blood compartment |
| $k_{on}$ | ESA-EpoR association rate / on-rate |
| $k_{off}$ | ESA-EpoR dissociation rate / off-rate |
| $K_D$ | ESA-EpoR dissociation constant ($k_{off}/k_{on}$) |
| $k_t$ | Ligand-independent receptor turnover rate |
| B$_{max}$ | Number of ESA binding sites per cell / per patient |
| $k_e$ | ESA-EpoR complex internalization constant |
| $k_{ex}$ | ESA and EpoR recycling constant |
| $k_{di}$ | Intracellular ESA degradation constant |
| $k_{de}$ | Extracellular ESA degradation constant |
| $k_{Hb\_pro}$ | Hemoglobin production constant by the ESA-EpoR complex |
| $K_{Hb\_deg}$ | Hemoglobin degradation constant (net loss of hemoglobin) |

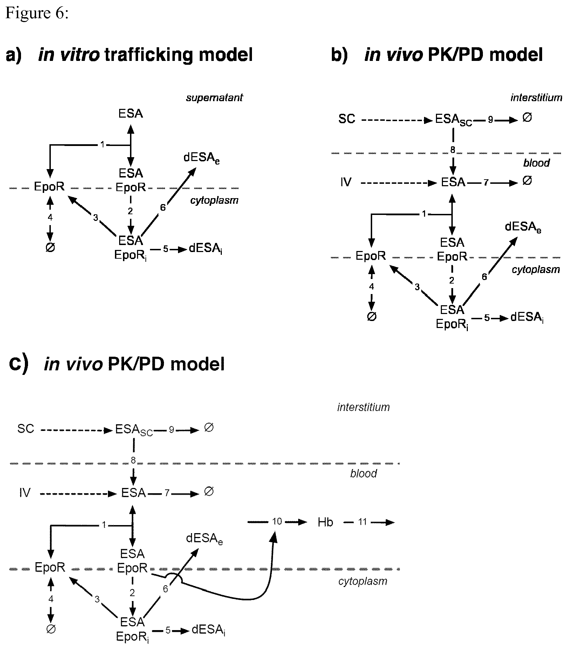

The models disclosed in the present application are based on the following ordinary differential equations with reference to figure 6. This model describes the following reaction scheme which is based on prior biological knowledge. The ESA binds reversibly ($k_{on}$ respectively $k_{off}$) to the Epo receptor (EPO-R) which is exposed on the cell surface. Thereby, the ESA-receptor complex gets activated and can induce phosphorylation of downstream signaling molecules like STAT5. The ESA-receptor complex is then internalized ($k_e$) into intracellular receptor pools where ESA is either exported ($k_{ex}$) or degraded ($k_{de}$ and $k_{di}$) and the receptor can trans-locate back to the membrane ($k_{ex}$). In addition, a ligand independent turnover ($k_t$) of EpoR ensures that the cell is sensitive for a broad range of ligand concentrations. In the equations [] denote concentrations of the respective components. These are, EpoR or EPO-R is the EPO receptor, ESAEpoR is the complex of ESA bound to the EPO-R. ESAEpoRi is the internalized complex. dESA is degraded ESA, either cell-internally (dESAi) or extracellular (dESAe). The equations are:

$$(1.1) \quad \frac{d[ESA]}{dt} = -k_{on} \cdot [ESA] \cdot [EpoR] + k_{off} \cdot [ESAEpoR] + k_{ex} \cdot [ESAEpoRi]$$

$$(1.2) \quad \frac{d[EpoR]}{dt} = -k_{on} \cdot [ESA] \cdot [EpoR] + k_{off} \cdot [ESAEpoR] + k_t \cdot B_{max} - k_t \cdot [EpoR] +$$

$$k_{ex} \cdot [ESAEpoRi]$$

$$(1.3) \quad \frac{d[ESAEpoR]}{dt} = k_{on} \cdot [ESA] \cdot [EpoR] - k_{off} \cdot [ESAEpoR] - k_e \cdot [ESAEpoR]$$

$$(1.4) \quad \frac{d[\text{ESAEpoRi}]}{dt} = k_e \cdot [\text{ESAEpoR}] - k_{ex} \cdot [\text{ESAEpoRi}] - k_{di} \cdot [\text{ESAEpoRi}] - k_{de} \cdot [\text{ESAEpoRi}]$$

$$(1.5) \quad \frac{d[\text{dESAi}]}{dt} = k_{di} \cdot [\text{ESAEpoRi}]$$

$$(1.6) \quad \frac{d[\text{dESAe}]}{dt} = k_{de} \cdot [\text{ESAEpoRi}].$$

For the model simulating the *in-vivo* patient situation this model is extended resulting in system of seven coupled ordinary differential equations (ODE). The expanded model in figure (6b) describes the situation including the blood and interstitium compartments. Intraveneous ESA is either cleared in the blood compartment ($k_{clear}$) or binds to the EPO-R ($k_{on}$, $k_{off}$). Subcutaneous applied ESA ($\text{ESA}_{SC}$) is transported to the blood compartment ($k_{sc\_out}$) or saturable cleared in the interstitial compartment ($k_{sc\_clear\_sat}$). The non-linear dynamic pharmacokinetic ESA-EPO-R pathway model:

$$(2.1.) \quad \frac{d[\text{ESA}_{SC}]}{dt} = -k_{sc\_clear} \cdot \frac{[\text{ESA}_{SC}]}{(k_{sc\_clear\_sat} + [\text{ESA}_{SC}])} - k_{sc\_out} \cdot [\text{ESA}_{SC}]$$

$$(2.2.) \quad \frac{d[\text{ESA}]}{dt} = k_{sc_{out}} \cdot [\text{ESA}_{SC}] - k_{clear} \cdot [\text{ESA}] - k_{on} \cdot [\text{ESA}] \cdot [\text{EpoR}] + k_{off} \cdot [\text{ESAEpoR}] + k_{ex} \cdot [\text{ESAEpoRi}]$$

$$(2.3.) \quad \frac{d[\text{EpoR}]}{dt} = -k_{on} \cdot [\text{ESA}] \cdot [\text{EpoR}] + k_{off} \cdot [\text{ESAEpoR}] + k_t \cdot B_{max} - k_t \cdot [\text{EpoR}] + k_{ex} \cdot [\text{ESAEpoRi}]$$

$$(2.4.) \quad \frac{d[\text{ESAEpoR}]}{dt} = k_{on} \cdot [\text{ESA}] \cdot [\text{EpoR}] - k_{off} \cdot [\text{ESAEpoR}] - k_e \cdot [\text{ESAEpoR}]$$

$$(2.5.) \quad \frac{d[\text{ESAEpoRi}]}{dt} = k_e \cdot [\text{ESAEpoR}] - k_{ex} \cdot [\text{ESAEpoRi}] - k_{di} \cdot [\text{ESAEpoRi}] - k_{de} \cdot [\text{ESAEpoRi}]$$

$$(2.6.) \quad \frac{d[\text{dESAi}]}{dt} = k_{di} \cdot [\text{ESAEpoRi}]$$

$$(2.7.) \quad \frac{d[\text{dESAe}]}{dt} = k_{de} \cdot [\text{ESAEpoRi}].$$

Since the amount of hemoglobin (Hb) in a patients serum is directly correlated to the activity of ESA-EPO-R system, the invention may instead of determining the concentration of the ESA after initial administration of the ESA as a function of time, determine the Hb concentration, which is a standard parameter observed during anemia treatment. In this embodiment, the above model comprises the additional reactions of the production of Hb by the activated ESA-EPO-R ($k_{Hb\_pro}$) and the patient specific degradation of Hb ($k_{Hb\_deg}$).

In this case the model includes the additional ODE:

$$(2.8.) \quad \frac{d[Hb]}{dt} = k_{Hb_{pro}} \cdot [\text{ESAEpoR}] - k_{Hb_{deg}} \cdot [Hb]$$

For both models the dissociation constant of $K_D$ is defined as

$$(3.1) \quad K_D = k_{off}/k_{on}$$

In these models $B_{max}$ is the initial number of binding sites for ESA.

Further explanation of the equations is provided in the example section and figure 6.

The values for the respective concentrations of elements and the all constants used in the above equations can be determined experimentally using, for example, a method known to the skilled person or the methods provided herein below in the example section.

In accordance with the present invention, a clinically safe dose of an ESA is a dose approved by the authorities for the treatment of anemia.

In the herein described methods clearance rate of an ESA in the serum of a patient is determined. Preferably, and this holds true for all aspects and embodiments as described herein, the clearance rate (or change of concentration) of said ESA is determined based on the initial dose of ESA administered to a patient. Subsequent to the initial ESA administration, samples obtained from a patient can be analyzed for the remaining ESA concentration for at least one time point subsequent to the initial ESA treatment. Ideally, the ESA concentration is observed over several time points, for example 1 to 6 weeks, preferably 1 to 3 weeks, and includes at least 2, preferably 5, more preferably 7 to 10 independent measurements of ESA concentration at different time points. An example for an observation plan would be the administration of the ESA at day 0, and the subsequent measuring of the ESA concentration in the patient at days 1, 2, 3, 5, 7, 10 and 14. This may be adjusted depending on the clinical scenario. For the alternative embodiment of the invention regarding the calculation of initial ESA binding sites based on the observation of the change of Hb concentration in a patient, the same principle is applied.

In a certain embodiment of the invention the ESA is any ESA known to the skilled person, which includes in particular EPO biosimilars, but is preferably selected from the group of Epoetin alfa, Epoetin beta, Novel erythropoiesis stimulating protein (NESP) and Continuous erythropoietin receptor activator (CERA). CERA is preferred for the herein described invention.

Preferable the calculation is further based on the initial ESA dose, and the initial Hb concentration in the patient at the time the ESA was administered.

In context of the here described invention a patient is preferably a patient that is suffering from anemia in the context of a cancer disease or chemotherapy, the cancer disease preferably being a lung cancer such as non-small cell lung cancer (NSCLC).

In preferred embodiments the non-linear dynamic pharmacokinetic (PK) ESA-EPO-R pathway model is based on a system of the ordinary differential equations (ODE) as described above. In this context the invention seeks to obtain the initial number of ESA binding sites, which is $B_{max}$. $B_{max}$ is therefore predictive for or an approximation of the colony forming units erythroid (CFU-E).

The problem of the invention is additionally solved by a computer implemented method, preferably performed *in silico,* for stratifying an anemia patient who receives treatment with an erythropoiesis Stimulating Agents (ESA), wherein the patient is stratified into a high risk or low risk group of experiencing an adverse event upon continued treatment with the ESA, the method comprising the steps of::

(a) Obtaining the initial administered ESA dose of said patient and Hemoglobin degradation rate [Hb degr] of said patient (see above how to obtain a patient's Hb degradation rate),
(b) Obtaining the concentration of said ESA in a serum sample of said patient at at least one second time point after the initial administration of said ESA to said patient.
(c) Determining the concentration rate of said ESA as a function of time in said patient
(d) Calculating based on a non-linear pharmacokinetic (PK) ESA-EPO-R model as described herein above and the concentration rate of said ESA in said patient the initial number of ESA binding sites [EpoR] in said patient,
(e) Calculating from (i) the patient's Hb degradation rate, (ii) the number of ESA binding sites, and (iii) the last ESA dose administered, or ESA dose planned to be administered, to said patient [ESA], an accumulated risk factor [aRF], and

(f) Stratifying the patient into a high risk or low risk group of experiencing an adverse event upon continued treatment with the ESA according to the [aRF].

The [aRF] is determined by a linear combination of (i) the individual Hb degradation rate, (ii) the number of ESA binding sites, and (iii) the last ESA dose administered, or ESA dose planned/calculated to be administered. Preferably, according to the equation A as described herein:

In preferred aspects the factors of the above equation A are B0=2.3518, B1=-2.5840, B2=-0.3957, and B3=-0.1374. Preferably, the factors may vary upon situation, but not more than +/- 20%, 1%, 10%, and preferably not more than 5% from the above indicated value. These are particularly useful in the event the patient is treated with CERA and suffers from NSCLC.

In preferred embodiments of the invention, a patient is at a high risk to experience an adverse event if [aRF] is 0.1 or higher, preferably 0.15 or higher, or 0.17 or higher, and most preferably wherein [aRF] is > about 0.18.

Yet another aspect of the invention provides a computer-readable storage medium having computer-executable instructions stored, that, when executed, cause a computer to perform a computer implemented method according to the present invention.

However, preferred is the above method wherein said organism is a patient, preferably a human patient, or wherein said cell is a cell endogenously expressing the EPO-R receptor, such as a red blood cell precursor cell, or a tumor cell.

Yet another aspect of the invention provides a computer-readable storage medium having computer-executable instructions stored, that, when executed, cause a computer to perform a computer implemented method according to the present invention.

In preferred embodiments of all aspects of the invention the $K_D$ of the ESA is about 16 pM for Epoetin alfa, about 17 pM for Epoetin beta, about 789 pM for NESP and about 982 pM for CERA.

The term "about" in relation to a numerical value x is optional and means, for example, $x\pm20\%$, $x\pm15\%$, $x\pm10\%$, $x\pm5\%$, or most preferably $x\pm2\%$. Preferably, all numerical values in the present disclosure allow for a variation of $x\pm5\%$, where x is the numerical value.

In a further aspect of the present invention there is provided an Erythropoiesis Stimulating Agent (ESA) for use in the treatment of anemia, the treatment comprising the steps of

(a) Determining whether a patient suffering from anemia has a low risk or high risk of an adverse event upon ESA treatment using a method according to the herein disclosed invention, and
(b) If the patient has a low risk of an adverse event upon ESA treatment, administering to said patient a therapeutically effective amount of an ESA.

In a preferred embodiment, the adverse event is a fatal outcome such as death of the patient.

The non-linear dynamic Hb ESA-EPO-R pathway model used in this aspect takes into account the additional reactions of the production of Hb based on the active ESA-EPO-R complex and a patients individual Hb degradation.

Also provided in context of the invention is an ESA for use in the treatment of anemia in a patient, wherein the patient has a low risk of an adverse event upon continued treatment with the ESA. The risk is determined according to a diagnostic method for stratification as described herein elsewhere. The treatment of anemia according to the invention in some embodiments comprises the obtaining blood samples of said patient in the first 1 to 5 weeks of the ESA treatment, and calculating therefrom the patient's individual risk of an adverse event upon continued treatment with the ESA, as disclosed herein elsewhere.

In another aspect there is also provided a method for treating a patient suffering from anemia associated with a cancer disease, chemotherapy induced anemia, or anemia associated with chronic inflammation, the method comprising the steps of

(a) Administering to the patient a low ESA dose for the first 1 to 5 weeks, preferably 3 weeks,
(b) Obtaining at least two samples from the patient during the first 1 to 5 weeks, preferably 3 weeks,
(c) Determining from said at least two samples the patient's risk of an adverse event upon continued treatment with the ESA,
(d) Administering to the patient ESA after the first 1 to 5 weeks if the patient is at low risk of an adverse event upon continued treatment with the ESA, or
(e) Administering to the patient a blood transfusion after the first 1 to 5 weeks if the patient is at high risk of an adverse event upon continued treatment with the ESA.

In preferred embodiments, in step (c), the patient's risk of an adverse event upon continued treatment with the ESA is determined by a method for stratification as described herein before.

The term "treatment" as used herein covers any treatment of a disease or condition (e. g., anemia) in a mammal, particularly a human, and includes: (i) preventing the disease or condition from occurring in a subject which may be predisposed to the disease but has not yet been diagnosed as having it; (ii) inhibiting the disease or condition, i. e. arresting its development; or (iii) relieving the disease or condition, i. e. causing its regression or the amelioration of its symptoms.

As used herein, the term "therapeutically effective amount" refers to that amount of a polymer-modified synthetic erythropoiesis stimulating protein which, when administered to a mammal in need thereof, is sufficient to effect treatment (as defined above), for example, as inducer of red cell production, an anti-anemia agent, etc. The amount that constitutes a "therapeutically effective amount" will vary depending on the ESA, the condition or disease and its severity, and the patient to be treated, its weight, age, gender, etc., but may be determined routinely by one of ordinary skill in the art with regard to contemporary knowledge and to this disclosure.

Administration of the ESA of the invention may be performed via any accepted systemic or local route known for the respective ESA, for example, via parenteral, oral (particularly for infant formulations), intravenous, nasal, bronchial inhalation (i. e., aerosol formulation), transdermal or topical routes, in the form of solid, semi-solid or liquid or. aerosol dosage forms, such as, for example, tablets, pills, capsules, powders, liquids, solutions, emulsion, injectables, suspensions, suppositories, aerosols or the like. The erythropoiesis stimulating agents of the invention can also be administered in sustained or controlled release dosage forms, including depot injections, osmotic pumps, pills, transdermal (including electrotran-sport) patches, and the like, for the prolonged administration of the polypeptide at a predetermined rate, preferably in unit dosage forms suitable for single administration of precise dosages. The compositions will include a conventional pharmaceutical carrier or excipient and a protein antagonist or agonist of the invention and, in addition, may include other medicinal agents, pharmaceutical agents, carriers, adjuvants, etc. Carriers can be selected from the various oils, including those of petroleum, animal, vegetable or synthetic origin, for example, peanut oil, soybean oil, mineral oil, sesame oil, and the like. Water, saline, aqueous dextrose, and glycols are preferred liquid carriers, particularly for injectable solutions. Suitable pharmaceutical carriers include starch, cellulose, talc, glucose, lactose, sucrose, gelatin, malt, rice, flour, chalk, silica gel, magnesium stearate, sodium stearate, glycerol monostearate, sodium chloride, dried skim milk, glycerol, propylene glycol, water, ethanol, and the like. Other suitable pharmaceutical carriers and their formulations are described in"Remington's Pharmaceutical Sciences"by E. W. Martin.

Another aspect of the invention further An Erythropoiesis Stimulating Agent (ESA) for use in the treatment of anemia in a subject, the treatment comprising the steps of

(a) Determining or providing hemoglobin concentrations in the subject from at least two separate time points and calculating therefrom a subject specific hemoglobin degradation rate,
(b) Determining the present hemoglobin concentration in the subject,
(c) Calculating from the subject specific hemoglobin degradation rate and the hemoglobin concentration in the subject the number of ESA binding site in the patient and the dosage of an ESA sufficient to treat the anemia in the subject using a non-linear dynamic pharmacokinetic (PK) hemoglobin (Hb) ESA-EPO-R pathway model,
(d) Determining from (i) the patient's Hb degradation rate, (ii) the patients number of ESA binding sites, and (iii) the calculated ESA dose sufficient to treat the anemia in the patient [ESA], an accumulated risk factor [aRF],
(e) Stratifying the patient into a high risk or low risk group of experiencing an adverse event upon continued treatment with the ESA according to the [aRF],
(d) Administering to the subject the calculated dosage of the ESA as determined in (c) if the patient is in a low risk group of experiencing an adverse event upon continued treatment with the ESA,
(e) Optionally, monitoring the hemoglobin concentration in the subject after administration of the ESA and adjusting the next dosage of the ESA by repeating steps (b) to (d).

In some embodiments the above method comprises the alternative steps:

(d') not administering to the subject an ESA, and optionally administering to the subject a blood transfusion.

The present invention will now be further described in the following examples with reference to the accompanying figures and sequences, nevertheless, without being limited thereto. For the purposes of the present invention, all references as cited herein are incorporated by reference in their entireties. In the Figures:

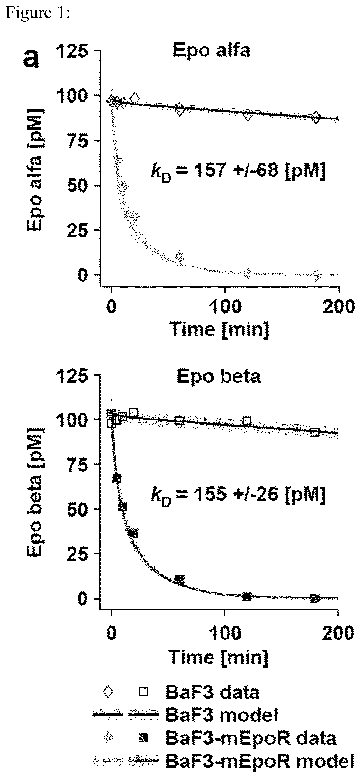

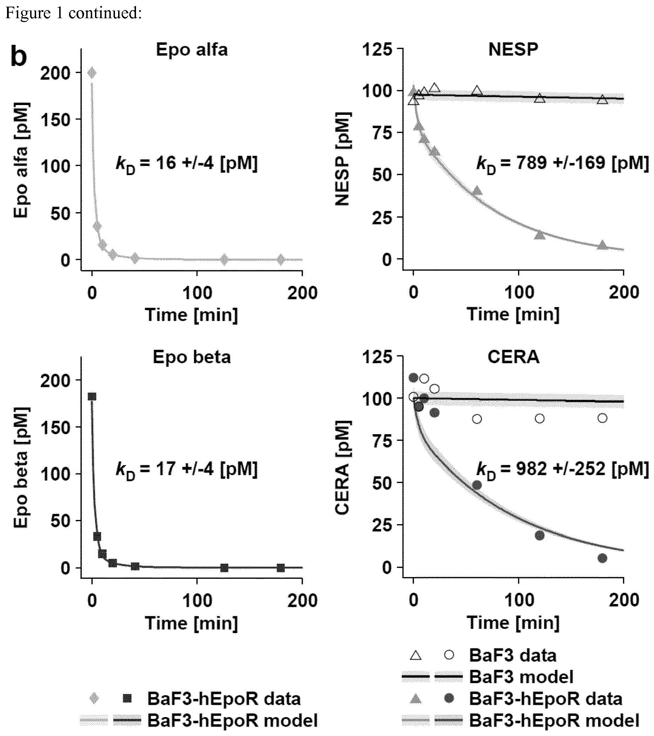

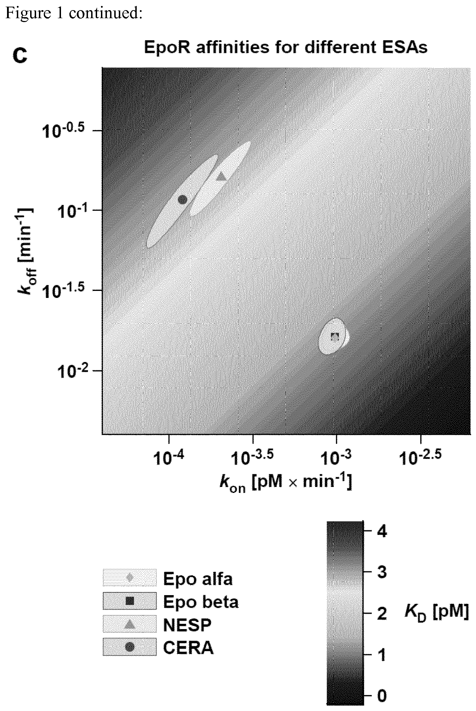

**Figure 1:** **Characterization of ESA binding properties based on the determination of ligand depletion and the ESA-EpoR mathematical model.** Parental BaF3 cells (BaF3) and BaF3 stably expressing the murine EpoR (BaF3-mEpoR) were incubated with 100 pM Epo alfa or 100 pM Epo beta. At the indicated times the supernatant was removed and the concentration of Epo was quanti-fied by an ELISA assay. Based on this data the association rate $k_{on}$, the dissoci-ation rate $k_{off}$ and the number of ESA binding sides at the cellular surface ($B_{max}$) were estimated by the ESA-EpoR mathematical model and the ESA-specific dissociation constant $K_D$ ($k_{off}/k_{on}$) was calculated. (a) BaF3 cells and BaF3 stably expressing the human EpoR (BaF3-hEpoR) were incubated with Epo alfa, Epo beta, NESP and CERA. At the indicated times the supernatant was removed and the concentration of Epo was quantified by an ELISA assay. Based on this data the association rate $k_{on}$, the dissociation rate $k_{off}$ and the number of ESA binding sides at the cellular surface ($B_{max}$) were estimated by the ESA-EpoR mathematical model and the ESA-specific dissociation constant $K_D$ ($k_{off}/k_{on}$) was calculated. (b) Predicted by the ESA-EpoR mathematical model for each ESA the association rate $k_{on}$ was plotted against the dissociation rate $k_{off}$. The calculated ESA-specific dissociation constant $K_D$ for the hEpoR is indicated by symbols. Shaded areas around the symbols indicate the confidence interval of the $K_D$ ($k_{off}/k_{on}$). The heatmap displays the values of the $K_D$.

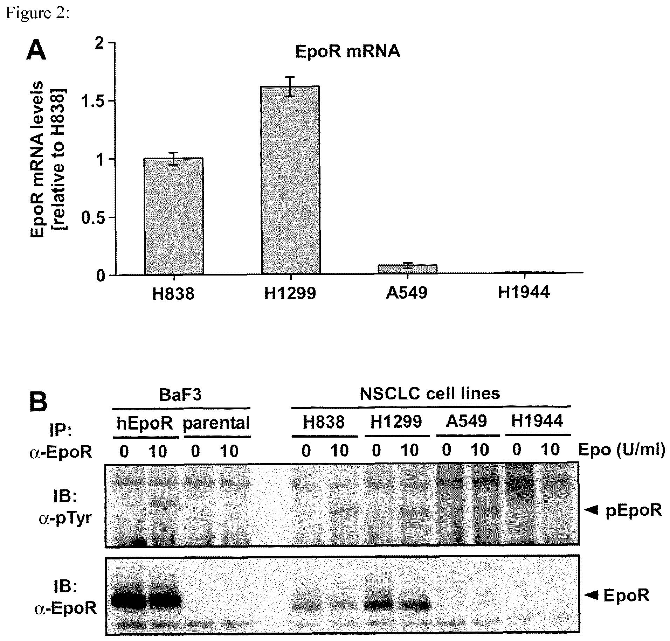

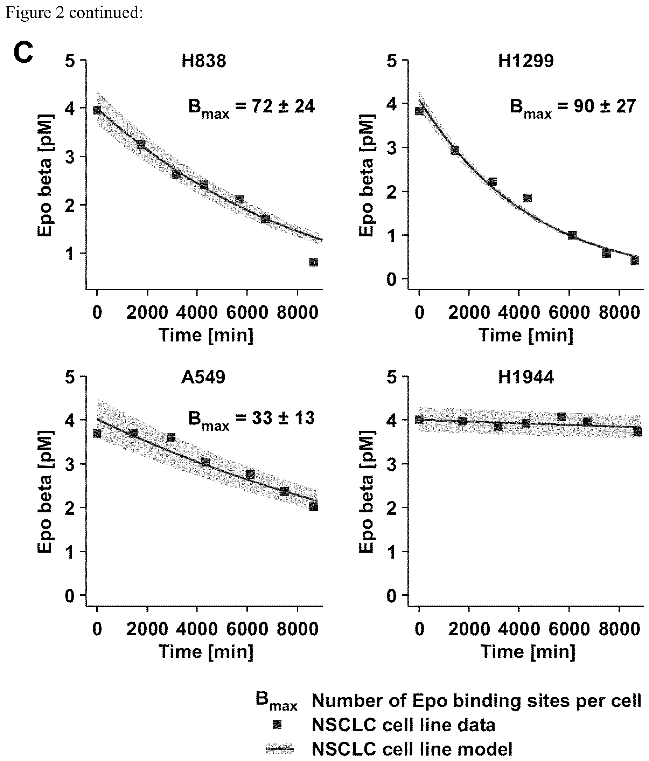

**Figure 2:** **Presence of a functional EpoR on human lung cancer cell lines.** (a) Total mRNA was extracted from the NSCLC cell lines H838, H1299, A549 and H1944 and the expression of the EpoR mRNA was determined by qRT-PCR. The EpoR mRNA expression in H838 cells was used as reference. (b) BaF3 cells and BaF3-hEpoR as well as the indicated NSCLC cell lines were stimulated with 10 U/ml of Epo beta for 10 min or were left untreated and were lysed. The abundance of the phosphorylated EpoR (pEpoR) and the total EpoR was determined by immunoprecipitation (IP) and quantitative immunoblotting (IB). The experiment was performed in biological triplicates and one representative immunoblot is shown. (c) The NSCLC cell lines H838, H1299, A549 and H1944 were stimulated with 4 pM of Epo beta and the Epo depletion kinetics was determined by an ELISA assay up to 8000 min incubation time. The ESA-EpoR mathematical model was employed to describe the depletion kinetics in all analyzed NSCLC cell lines and to determine the number of ESA binding sites / cell ($B_{max}$).

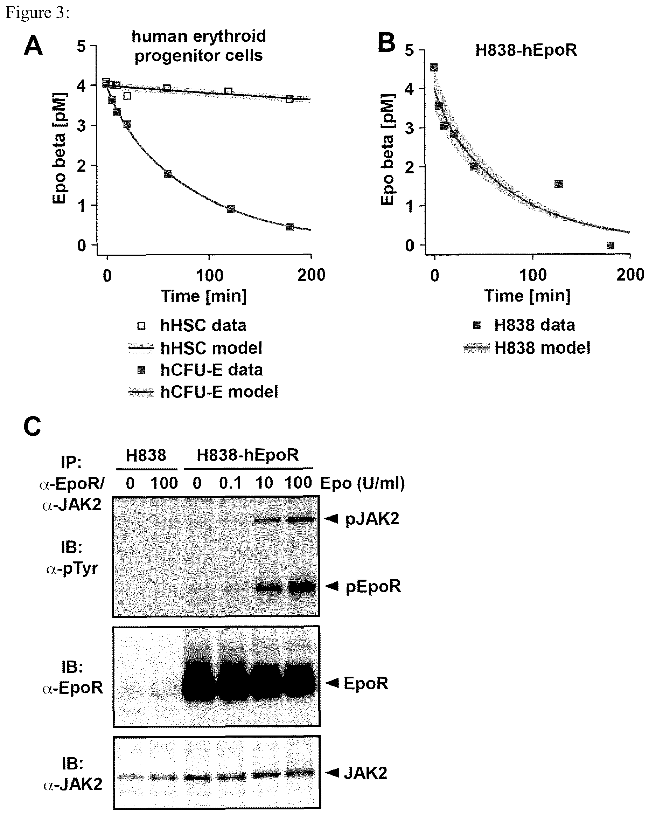

**Figure 3:** **H838-EpoR cells can serve as a model for human CFU-E cells concerning EpoR levels** (a) Human hematopoietic stem cells (hHSC) from cord blood were isolated and differentiated to human CFU-E (hCFU-E) as described. hCFU-E and hHSC cells that served as negative control (a) as well as NSCLC cell line H838 stably transduced with hEpoR (H838-EpoR) (b) were stimulated with 4 pM of Epo beta and time-resolved analysis of the depletion kinetics was monitored via ELISA assay over the time period of 200 min (experimental data - dots). The model could describe the depletion kinetics (model - solid line) and estimate KD and Bmax values. (c) Quantitative immunoblot demonstrating overexpression level of human EpoR in H838-hEpoR cells compared to parental H838. Functionality of EpoR is shown by Epo-induced phosphorylation of receptor and JAK2.

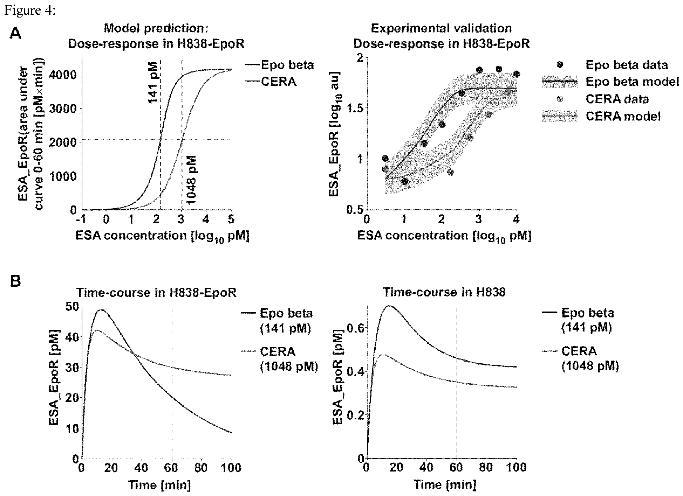

**Figure 4:** **CERA preferentially activates cells with high EpoR expression** (a) Model based prediction of differential dose response for EpoR activation in H838-hEpoR by different ESAS (left panel). Blue and red lines correspond to Epo beta and CERA respectively. Dashed lines indicated the EC50 of each ESA in the activation of the erythroprogenitors, 141 pM and1048 pM for Epo beta and CERA respectively. Right panel represents the validation of the model prediction. Epo beta and CERA activates EpoR in a very different range of concentrations. H838-hEpoR cells were stimulated during 10 minutes with increasing concentrations of each ESA. Cells were lysed, EpoR immunoprecipitated and blotted against total and phosphorylated form. Blue circles represent experimental data upon Epo beta stimulation. Red circles represent experimental data corresponding to CERA stimulation. Solid lines are the activation trajectories predicted by the model. (B) Left panel represents the model based prediction of the integral EpoR activation by each EC50 during 60 minutes. Area under the curve shows no significant difference between Epo beta and CERA activation in H838-EpoR, Right panel shows the model based prediction of the integral EpoR activation by each EC50 during 60 minutes in H838. In this case the area under the curve indicates a probable lower activation of EpoR by CERA in comparison with Epo beta.

**Figure 5:** **Differential pharmacokinetic behavior of CERA among healthy and NSCLC subjects.** (a) Pharmacokinetic behavior of increasing CERA concentrations in healthy volunteers. Colored circles are the mean values of CERA concentrations in serum, determined by ELISA assay. Solid lines represent the trajectories predicted of the CERA clearance for the given concentrations and the experimental data. (B) Pharmacokinetic behavior of increasing CERA concentrations in NSCLC patients in stage III or IV. Colored circles are the mean values of CERA concentrations in serum, determined by ELISA assay. Solid lines represent the trajectories predicted of the CERA clearance for the given concentrations and the experimental data. The different trajectories reported by the model, describes the experimental data and showed a reduction of

72%$\pm$16% in the CERA clearance capability of NSCLC patients. (c) Characterization and relative comparison of CERA clearance capability (% of CFU-E) of NSCLC patients and healthy subjects. The dashed line is the 100% clearance capability of CERA, which represents the normal capability of CERA clearance in healthy subjects. The pinky bars represent the number of NSCLC patients with a define % of CERA clearance capability compared to healthy subjects (individual PK data extracted from Hirsch et al 2007 clinical trial). The plot represents a general reduction of CFU-E population (% of CERA clearance capability) in NSCLC patients in comparison in comparison of the mean value in healthy subjects represented as 100%. It can be also notice different grades of reduction in the CFU-E population of NSCLC patients.

**Figure 6:** **Graphical representation of the basic and pharmacokinetic/pharmacodynamic mathematical model.** (a) the reactions 1 to 6 are 1:Binding/unbinding of ESA to the Epo receptor (EpoR). The kon/koff rate constants of the binding/unbinding reaction are ESA specific and can be fully characterized using the trafficking model and the respective depletion data. 2: ESA-EpoR complex internalization. 3: Recycling to the cell membrane and dissociation of the internalized ESA-EpoR complex. 4: Production/degradation of EpoR at the cell membrane. The production/degradation reactions are in equilibrium defining a certain, cell type (a) / patient (b) specific amount of receptors at the cell surface characterized by Bmax parameter. 5: Degradation of internalized ESA-EpoR complex. 6: Degradation and release of internalized ESA-EpoR complex; (b) additional reactions 7 to 9 are 7: Clearance in the blood compartment, 8: Transport into blood compartment, 9: Saturable clearance in the interstitial compartment. (c) Calculation of $B_{max}$ based on the Hb levels further includes the reactions 10: Production of Hb triggered by the activated receptor complex, and 11: depletion of Hb in the blood of an individual.

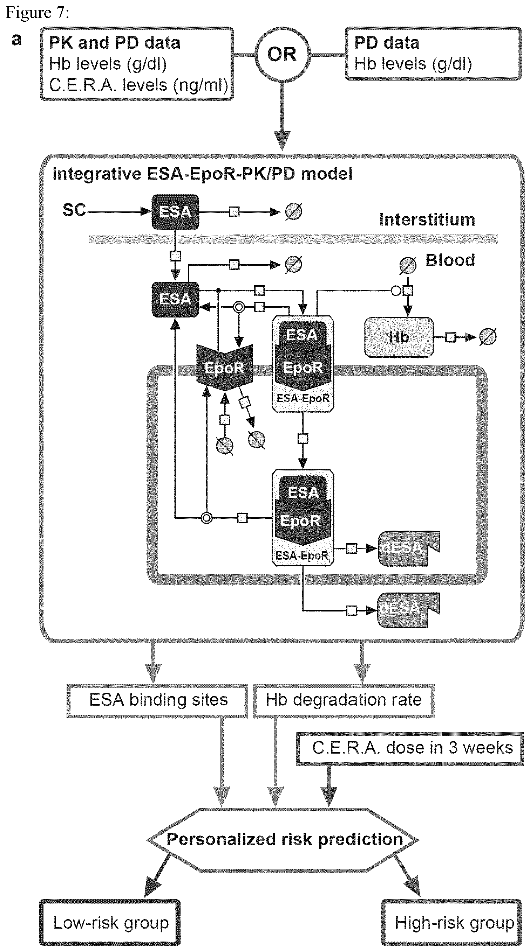

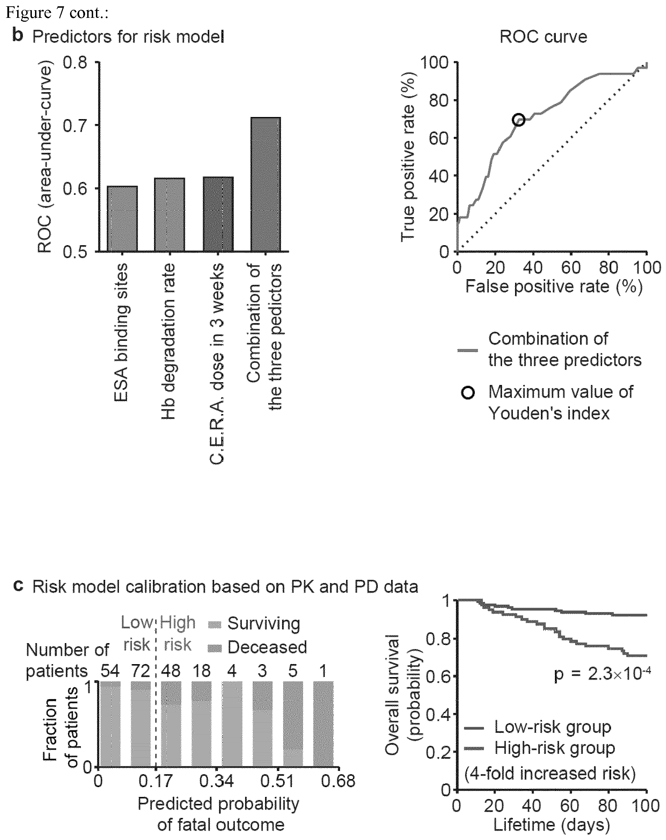

**Figure 7:** **Link of the ESA-EpoR mathematical model to risk prediction.** a, Flowchart depicting patient-specific risk prediction by the ESA-EpoR-PK/PD model based on PK/PD or PD data. b, Left panel represents the correlation by logistic regression of the model-inferred patient-specific parameters (ESA binding site and Hb degradation rate) and ESA treatment or the combination thereof with fatal outcome in NSCLC patients. Right panel displays the ROC curve corresponding to the combination of the three parameters as risk predictors of fatal outcome. c, Left panel corresponds to patients grouped based on their predicted probability of fatal outcome. Green and orange indicate the fraction of surviving and deceased patients. Dashed line indicates the risk threshold according to the criteria established in (b). Right panel displays the overall patients survival in a Kaplan-Meier plot, blue and red colors corresponds to the predicted low and high risk of fatal outcome for each group of patients. Cox-proportional hazard-ratio was used to calculate a 4-fold increase in risk (p<0.01). The calibration of the model for the risk stratification was performed on the PK/PD datasets (N=205) in the NSCLC clinical trials (NCT00072059 and NCT00327535).

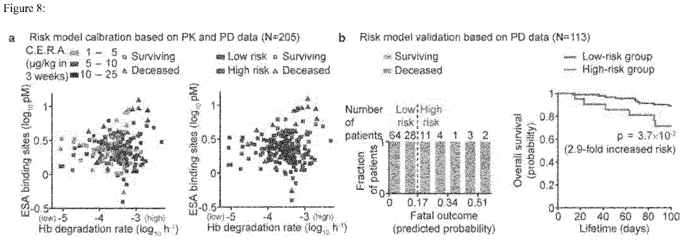

**Figure 8:** **Model-based response optimization and risk prediction. a,** Patient stratification based on individual estimations of ESA binding sites and Hb degradation rate in both NSCLC clinical trials (NCT00072059 and NCT00327535)[40,41]. Patients who died during the trial are depicted as triangles, and survivors as squares. In the left panel, the colour code indicates the ESA dose given in the first three weeks. In the right panel, red and blue colours indicate the classification of patients in high risk and low risk of fatal outcome. **b,** Representation of an independent dataset of the NCT00072059[40] clinical trial for which only PD values were available. Left panel corresponds to patient groups based on their predicted probability of fatal outcome. Green and orange indicate the fraction of patients that survived or died. Dashed line indicates the risk threshold according to the criteria established in Supplementary Fig. 38. Overall survival is displayed as a Kaplan-Meier plot (right panel), blue and red colours correspond to the predicted low and high risk group. The Cox proportional hazard model was used to determine a 2.9-fold increase in risk (p<0.05).

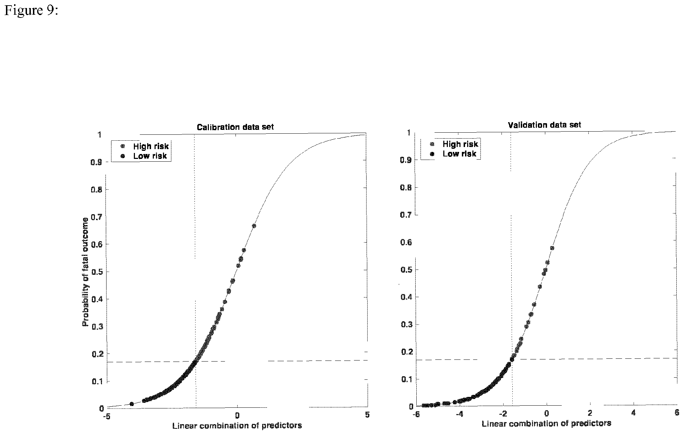

**Figure 9:** **Plot of relative risk of an adverse eventagainst the accumulated risk factor aRF;** a. calibration dataset b. validation dataset.

EXAMPLES

**Materials and Methods**

Plasmids and reagents.

Retroviral expression vectors were pMOWS-puro (Ketteler et al., 2002). The generation of hemagglutinin (HA)-tagged murine Epo receptor (pMOWS-HA-mEpoR) and of HA-tagged human EpoR (pMOWS-HA-hEpoR) was performed as described previously (Becker et al., 2010). Cells were either treated with Epo alfa (Cilag-Jansen), Epo beta (Roche), NESP (Amgen), or CERA (Roche) at indicated concentrations.

Cell culture and transfection.

Human lung adenocarcenoma cell lines A549, H838, H1299, H1944, H1650, H1975 and H2030 were purchased by ATCC and cultivated in Dulbecco's modified Eagle's Medium (DMEM, Lonza) supplemented with 10% fetal calf serum (FCS, Gibco) and 1% penicillin/streptomycin (Invitrogen). The Phoenix eco and Phoenix ampho packaging cell lines (Kinsella & Nolan, 1996) were cultured in DMEM (Gibco) supplemented with 10% FCS and 1% penicillin/streptomycin. BaF3 cells (Palacios & Steinmetz, 1985) were cultured in RPMI-1640 (Invitrogen) including 10% FCS and supplemented with 10% WEHI conditioned medium as a source of IL-3. For the EpoR overexpressing cell lines H838 (H838-hEpoR) and BaF3 (BaF3-mEpoR and BaF3-hEpoR) 1,5 $\mu$g/ml puromycin (Sigma) was added to the respective medium.

To obtain hCFU-E cells, CD34+ cells were sorted by MACS (CD34-Multisort Kit, Miltenyi) from umbilical cord blood of healthy donors after written consent. CD34+ cells were expanded using Stem Span SFEM II supplemented with Stem Span CC110 (both StemCell Technology). After seven days of expansion cells were either washed extensively using IDMEM (Gibco) to remove cytokines and to initiate differentiation or cells were used for depletion experiments. For differentiation cells were cultivated in Stem Span SFEM II supplemented with 10 ng/ml IL-3 (R&D Systems), 50 ng/ml SCF (R&D Systems) and 6 U/ml Epo alpha (Cilag-Jansen) as published by Miharada 2006. After 4 days of cultivation in this media hCFU-E were harvested to perform depletion experiments. All cells were cultured at 37 °C with 5% CO2 incubation.

Transfection of Phoenix eco and Phoenix ampho cells was performed by calcium phosphate precipitation. Transducing supernatants were generated 24 h after transfection by passing through a 0,45 $\mu$m filter and supplemented with 8 $\mu$g/ml polybrene (Sigma). Stably transduced BaF3 cells expressing HA-tagged murine EpoR (BaF3-mEpoR cells) or HA-tagged human EpoR (BaF3-hEpoR cells) or H838 cells expressing HA-tagged human EpoR (H838-hEpoR cells) were selected in the presence of 1,5 $\mu$g/ml puromycin (Sigma) 48 h after transduction. Surface expression of EpoR in BaF3 and H838-hEpoR cells was verified by Flow cytometry analysis.

Flow cytometry.

EpoR surface expression was verified by flow cytometry. Therefore H838-hEpoR cells were gently detached with Cell Dissociation Solution (Sigma) according to the manufacturer's instructions. BaF3-EpoR and H838-hEpoR cells were stained with anti-HA antibody (Roche) diluted 1:40 in 0,3% PBS/BSA for 20 min at 4 °C. Followed by washing of cells with 0,3% PBS/BSA and incubation of secondary Cy5-labeled antibody against rat (Jackson Immuno Research), diluted 1:100 in 0,3% PBS/BSA, for 20 min at 4 °C in the dark. After washing samples with 0,3% PBS/BSA, propidium iodide (BD Biosciences) was added to exclude dead cells. Canto II (BD Bioscience) was used for sample analysis.

Depletion experiments and ELISA

ESA depletion experiments were conducted in NSCLC tumor cell lines, BaF3, BaF3-mEpoR, BaF3-hEpoR, hCFU-E, hHSC cells. Tumor cells were seeded in 6 well-plates (TPP 92006) at a cellular concentration of $4\times105$ cells in 3 ml of proliferating media (DMEM supplemented with 10% FCS and 1%). Cells were kept at 37°C, 95% H2O and 5% CO2 during three days. On the third day cells were washed with DMEM (1% penicillin/streptomycin and 1mg/ml BSA) and left them starving in 1 ml of washing media during 12 hours. Cells were stimulated with Epo alfa/beta within the indicated times and concentrations of the depletion plots. After the incubation time, media was recovered and kept at -80°C till the conclusion of the experiment, cells were trypsinized and counted by hemoytometer chamber. Once the experiment was concluded ESAs concentration was measured by ELISA (Quantikine IVD ELISA Kit, R&D DEP00).

The experimental setting for the depletion measurements was different in the suspension cells; BaF3-hEpoR, BaF3-mEpoR, BaF3, hCFU-E and hHSC. In the transduced BaF3 cells, the experiments were conducted in between 9-14 days of selection with puromicin (1,5 $\mu$g/ml). Cells were washed three times in RPMI by centrifugation 5 minutes at $212 \times$ g, and starved 3 hours in RPMI (1% penicillin/streptomycin and BSA 1mg/ml) at a concentration of $1\times106$ cells/ml. After the starvation period cells were adjusted to a final concentration of $40\times106$ cells/ml in 350 $\mu$l at 37°C and 900rpm in a Thermomixer compact of Eppendorf. Cells were stimulated by ESA during the indicated times in the plot and centrifuged during 5 minutes, at 4°C and 2500rpm. Supernatant was removed and kept at -80°C. ESAs measurements were performed by ELISA (Quantikine IVD ELISA Kit, R&D DEP00). ESAs depletion measurements were conducted in the same way in hCFU-E and hHSC with the only difference of the cell concentration $30\times106$ cells/ml, and the used media (Stem Span SFEM II).

Immunoprecipitation and quantitative immunoblotting

For analysis of phosphorylated and total proteins human lung adenocarcenoma cell lines as well as H838-hEpoR cell line were seeded, cultivated for 72 h, starved for 3 h in DMEM with 1% penicillin/streptomycin, 2 mM L-glutamine (Gibco) and 1 mg/ml BSA and then stimulated with Epo beta or CERA at indicated concentrations for 10 min. Prior to experiments BaF3 cells were washed and resuspended in serum-depleted RPMI-1640 supplemented with 1% penicillin/streptomycin and 1 mg/ml BSA and starved for 3 h. Afterwards the cells were harvested and aliquoted in a density of 20 x 106/ml and stimulated with Epo beta at indicated concentrations for 10 min.

The cells were lysed with 1.25x NP-40 lysis buffer (1.25% NP-40, 187.5 mM NaCl, 25 mM Tris pH 7.4, 12.5 mM NaF, 1.25 mM EDTA pH 8.0, 1.25 mM ZnCl2 pH 4.0, 1.25 mM MgCl2, 1.25 mM Na3VO4, 12.5% glycerol supplemented with aprotinin and AEBSF). The protein concentrations in lysates were measured using the colorimetric BCA protein assay kit (Pierce Protein Research Products). For Immunoprecipitation analysis the lysates (1500-2000 $\mu$g protein for lung adenocarcenoma cell lines, 400 $\mu$g protein for BaF3 cells) were supplemented with antibodies to EpoR (R&D, MAB 307), JAK2 (Upstate) or STAT5A/B (Santa Cruz, C17) and Protein A sepharose (GE Healthcare) and rotated over night by 4 °C. Immunoprecipitated proteins were separated by 10% SDS-PAGE and transferred to nitrocellulose membrane (0.2 $\mu$m pore, Schleicher & Schuell). For quantification purposes randomized non-chronological gel loading was performed (Schilling et al., 2005). For the detection of the phosphorylated proteins the blots were probed with mAbs specific for phosphotyrosine (pTyr) (Upstate, clone 4G10) and then with secondary horseradish peroxidase-coupled anti-mouse antibodies (Dianova). To remove antibodies, membranes were treated as described previously (Klingmüller et al., 1995) and subsequently incubated with pAbs for EpoR (Santa Cruz, C-20) and horseradish peroxidase-coupled anti-rabbit antibodies (Dianova). Detection was performed using ECL substrate (GE Healthcare). Immunoblot data were acquired with the CCD camera-based ImageQuant LAS 4000 (GE Healthcare) and quantification was performed with the ImageQuant TL version 7.0 software (GE Healthcare).

mRNA isolation, cDNA preparation and qPCR

For analysis of EpoR expression the cells were lysed and RNA extraction was performed using RNeasy Mini kit (Qiagen) according to the supplier's protocol. To obtain cDNA from RNA, the high-capacity cDNA reverse transcription kit (Applied Biosystems) was used according to manufacturer's instructions. Quantitative real-time PCR (qRT-PCR) analysis was performed using LightCycler 480 (Roche applied-Science). Samples were prepared with reagents of the LightCycler480 Probes Master Kit from Roche applied-Science. Specific primers were obtained from Eurofins MWG and universal probes (UPL) for TaqMan quantification of DNA from Roche applied-Science. Concentrations were normalized using the geometric mean of $\beta$-glucuronidase (GUSB) and esterase D (ESD). Primers targeting human EpoR: for-ward - ttggaggacttggtgtgtttc; reverse - agcttccatggctcatcct; ESD: forward - ttagatggacag-ttactccctgataa; reverse - ggttgcaatgaagtagtagctatgat; GUSB: forward - cgccctgcctatctgtattc; reverse - tccccacagggagtgtgtag.

Mass spectrometry analysis.

Cellular lysate were subjected to IP with a combination of two STAT5 antibodies, sc-1081 and sc-836 from Santa Cruz Biotechnology. Two IPs were pooled per lane. Proteins were separated by a 10% SDS-PAGE (GE Healthcare) in 1x Laemmli buffer (Laemmli 1970). Following coomassie staining with SimplyBlue™ SafeStain (Invitrogen) STAT5 gel bands were excised at approximately 90 kDa and cut into small pieces (1 mm3). Gel pieces were destained, reduced with DTT (dithiothreitol, SIGMA), alkylated with IAA (iodoacetamide, SIGMA) and digested with 0.3$\mu$g trypsin in 100mM NH4HCO3/5% acetonitrile buffer overnight. In-house produced one-source peptide/phosphopeptide ratio standards for STAT5A and STAT5B were added to the digests (Boehm 2014). Following a four-step peptide extraction performed sequentially with 100mM NH4HCO3/5% acetonitrile, acetonitrile, 5%formic acid, and acetonitrile, the samples were concentrated in a speedvac (Eppendorf) and desalted with C18 Ziptips (Millipore) using solutions based on water, acetonitrile and formic acid. Samples were analyzed by EASY-nLC 1000 (Thermo Scientific) coupled to a Q Exactive™ Hybrid Quadrupole-Orbitrap Mass Spectrometer (Thermo Scientific). As precolumn the inventorsused Acclaim PepMap 100, 75 $\mu$m x 2 cm, as analytical column the inventorsused Acclaim PepMap RSLC C18, 2 $\mu$m, 100 Å, 75 $\mu$m x 25 cm. Survey full scan MS spectra were acquired at resolution R = 70,000 and analyzed for the native and labelled STAT5 peptide and phosphopeptide pairs with Xcalibur 3.0.63 (Thermo).

The in vitro trafficking model (figure 6a) was extended to a pharmaco-kinetic/pharmacodynamics (PK/PD) model (figure 6b) by including blood and interstitium compartments and patient specific PK data obtained by either intravenous (IV) or subcutaneous (SC) injections of ESA/CERA. Additionally, the model provides the link between ESA bound to the EpoR (ESA_EpoR) and haemoglobin levels (Hb) measured in patients. The model consists of the following additional reactions:

7. Clearance in the blood compartment.
8. Transport into blood compartment.
9. Saturable clearance in the interstitial compartment.
10. Production of Hb triggered by the activated receptor complex.
11. Patient specific degradation of Hb.

The reaction rate equations are given by:

1. "$k_{on}$ * ESA * EpoR" and "$k_{off}$ * ESA_EpoR"
2. "$k_e$ * ESA_EpoR"
3. "$k_{ex}$ * ESA_EpoR_i"
4. "$k_t$ * Bmax" and "$k_t$ * EpoR"
5. "$k_{di}$ * ESA_EpoR_i"
6. "$k_{de}$ * ESA_EpoR_i"
7. "$k_{clear}$ * ESA"
8. "$k_{scout}$ * ESA_SC"
9. "$k_{scclear}$ * ESA_SC / ($k_{scclearsat}$ + ESA_SC)"
10. "$k_{hb\_pro}$ * ESA_EpoR"
11. "khb_deg * Hb"

Model calibration

For calibration of the model parameters, the inventors used the D2D software package (Raue et al. PloS ONE 2013) in MATLAB (Release 2012b, The MathWorks, Inc., Natick, MA, USA). In order to minimize the distance between the simulated model trajectories and the measured data, a maximum likelihood approach was applied. The inventors used a deterministic optimization algorithm combined with multiple starting points in the high dimensional parameter space to find the global optimum of the negative log-likelihood. As the parameter values can range over several orders of magnitude and are, by its biochemical definition, strictly positive, the optimization was performed in logarithmized parameter space. To account for the log-normally distributed measurement noise of protein time course data (Kreutz et al. Bioinformatics 2007), also the data were transformed onto the logarithmic scale and an additive error model was fitted simultaneously with the kinetic model parameters. (Raue et al. PloS ONE 2013)

The affinity parameters ($k_{on}$, $k_{off}$ or $k_{on}$ and $k_D$) and the number of binding sites ($B_{max}$) were estimated individually for each experimental condition, i.e. combination of ESA and cell type, as they depend on the biochemical properties of the ESA and on the EpoR expression level of the respective cell type.

The structural and practical identifiability of the parameters was analyzed using the profile likelihood approach as described by Raue et al. (Bioinformatics 2011). Furthermore, this method enabled the inventors to determine the parameter's confidence intervals and the uncertainties of the model predictions.

For risk prediction based on patient-specific parameters of the multi-scale mixed-effects ESA-EpoR-PK/PD model and the administered C.E.R.A. doses, logistic regression was used. Discrimination between low and high risk groups was based on Youden's index, maximizing specificity and sensitivity (Youden, W. J. Index for rating diagnostic tests. Cancer 3, 32-35 (1950)).

**Model based determination of ESA binding properties**

To assess the role of Epo and Epo derivatives in the context of lung cancer, it was essential to develop a reliable, quantitative assay that enables to determine the number of binding sides per cell and the specific binding properties of different human ESA (Epo alpha, Epo beta, NESP and CERA). The inventors utilized the inventor's knowledge that rapid ligand depletion is characteristic for the Epo-EpoR system (Becker et al 2010) and established a robust ELISA assay to monitor Epo removal from cellular supernatants.

As shown in Fig. 1a this enabled the inventors to accurately quantify the depletion of Epo alfa and Epo beta by murine BaF3 cells stably expressing the murine EpoR (BaF3-mEpoR) whereas parental BaF3 cells had no impact underscoring the specificity of the assay. These quantifications in combination with the inventor's dynamic pathway model of Epo-EpoR interactions (Becker et al 2010) enabled to calculate the dissociation constant $K_D$ (Fig. 1a) as well as the association rate $k_{on}$, the dissociation rate $k_{off}$ and the number of binding sides ($B_{max}$) for Epo alfa and Epo beta interaction with the murine EpoR.

The estimated $B_{max}$ was in good agreement with the results obtained by traditional saturation binding assays using radioactively labelled ligand, further validating the assay. To comparatively examine the binding properties of different ESAs for the human EpoR, the inventors measured ESA depletion by BaF3 cells stably expressing the human EpoR (BaF3-hEpoR) or parental BaF3 cells (Fig. 1b). The results showed that whereas Epo alpha and Epo beta are very rapidly depleted, depletion of NESP and CERA is moderate. The quantitative time-resolved data in combination with the inventor's dynamic pathway model of ligand-receptor interaction enabled the inventors to calculate that $K_D$ of Epo alpha and Epo beta, respectively, are with 16 and 17 pM very similar. However, for NESP the model indicates a $K_D$ of 789 pM and for CERA a KD of 982 pM suggesting for both Epo derivatives a much elevated dissociation constant.

Relating the $K_D$ of the different ESA to the respective association and dissociation rates as shown in Fig. 1c reveals that the association of NESP and CERA is much slower compared to Epo alpha and Epo beta whereas the dissociation rate is enhanced. Therefore by combining simple time-resolved quantification of the concentration of Epo in cell supernatants with the inventor's dynamic pathway model it was possible to reliably determine the binding properties of ESA and to show that the available ESA differ significantly in their properties to bind to the human EpoR.

**Presence of functional EpoR in NSCLC cell lines**

To determine the presence of a functional EpoR in lung cancer cells, the inventors first screened a panel of NSCLC cell lines for the presence of EpoR mRNA. Among these the inventorsidentified three adenocarcinoma NSCLC cell lines that showed significant levels of EpoR mRNA transcripts. As depicted in Fig 2a H838 and H1299 showed moderate expression levels of EpoR mRNA and A549 low levels. H1944 represent NSCLC cell lines with levels below the detection limit (Fig. 2a). Next evaluated was the expression of the EpoR protein in the four selected NSCLC cell lines as well as its functionality. Enrichment by immunoprecipitation and detection by immunoblotting revealed the presence of the EpoR protein in H838 and H1299 and at very low levels in A549, whereas it was absent in H1944 (Fig. 2b). In line with previous observations the overall expression level of EpoR protein was very low compared to BaF3-hEpoR.

Upon stimulation with Epo as expected the tyrosine phosphorylated form of the receptor was absent in parental BaF3 cells and H1944, but evident in H838, H1299 and A549 indicating the presence of a signaling competent, functional EpoR in these three NSCLC cell lines. To determine the binding properties of the EpoR expressed in the NSCLC cell lines, the inventors applied the depletion assay and showed (Fig. 2c) that Epo beta was depleted by the NSCLC cell lines harboring a functional EpoR, but not by the EpoR negative NSCLC cell line H1944 (Fig 1b). However, Epo beta depletion was much slower compared to BaF3-EpoR cells suggesting the presence of a significantly lower number of cell surface receptors. Accordingly, analysis of the time-resolved data with the dynamic pathway model revealed binding sides ranging from undetectable to 90 per cell (Fig. 2c and Table 2), yet the estimated $K_D$ was comparable to the estimates with BaF3-hEpoR. This shows that ligand depletion and signaling competent receptor is present on a subset of NSCLC cell lines.

**EpoR depletion kinetics in cells with high numbers of EpoR**

The main target of Epo treatment during anemia are erythroid progenitor cells at the colony forming units-erythroid (CFU-E) stage that express high levels of the EpoR. To quantify the cell surface expression of the EpoR on human CFU-E and characterize the binding properties, human CD34+ hematopoietic stem cells (hHSC) were prepared from human umbilical cord blood and differentiated to human CFU-E (hCFU-E). Time-resolved analysis of Epo beta depletion revealed rapid reduction of Epo beta from the supernatants of hCFU-E but not of hHSC that lack the EpoR (Fig. 3a). Model based analysis showed a $K_D$ comparable to BaF3-hEpoR and a $B_{max}$ of 365 binding sites per cell that was one order of magnitude lower compared to BaF3-hEpoR but one order of magnitude higher in comparison to the NSCLC cell line H838.

To examine whether some of the available ESA could have advantages in the tumor context due to the distinct binding properties, the inventors aimed at establishing a cell model system with elevated hEpoR expression levels mimicking the situation in hCFU-E as hCFU-E are only available at extremely limiting amounts. The inventors stably expressed the hEpoR in H838 (H838-hEpoR) and showed by enrichment using immunoprecipitation and immunoblotting that the expression of the EpoR was highly increased and the phosphorylated EpoR was substantially elevated (Fig. 3b). Depletion experiments and model-based analysis revealed binding properties rather similar to hCFU-E (Fig. 3c) establishing the H838-hEpoR cell line as suitable model system to examine the impact of different ESA on cells harboring high levels of the EpoR as observed in the hematopoietic system versus cells expressing low levels as in the tumor context.

**Identification of CERA as an ESA preferentially activating cells with high EpoR expression**

To compare the impact of ESA on tumor cells that express low levels of EpoR versus cells that display elevated EpoR levels such as H838-EpoR, model simulations were performed. As readout for EpoR signaling, the inventorscalculated the integral of ESA bound to the EpoR (ESA_EpoR) for the first 60 minutes after stimulation. First these stimulations were performed for different ESA concentrations and predicted the $EC_{50}$ for both Epo beta and CERA in cells with high EpoR levels (Fig. 4a). The model predicts that a 10-fold higher concentration of CERA is required for the same activation. This model prediction was experimentally validated in H838-EpoR cells by quantitative immunoblotting against phosphorylated EpoR.

Interestingly, the model predicted that the ESA concentrations that induce the same activation in cells with high EpoR levels act differently in cells with low levels of EpoR such as H838. As these cells deplete less Epo beta, Epo beta results in stronger activation than CERA in cells with low levels of EpoR (Fig. 4b). Experimentally this model prediction was validated in H838 cells by quantitative mass spectrometry against phosphorylated STAT5. Thus, CERA was identified as an ESA preferentially activating cells with high EpoR expression, such as H838-EpoR and hCFU-E cells, rather than cells with low EpoR expression, such as NSCLC cells.

**Determination of the number of CFU-E cells in healthy subjects and NSCLC patients by an integrated PK/PD model**

Having identified CERA as an ESA preferentially acting on cells with high EpoR levels, the inventors integrated the inventor's model with pharmacokinetic (PK) data to describe CERA dynamics in patients (the integrative (PK/PD) ESA-EpoR mathematical model ; see above). In a first step, the inventors analyzed mean PK values of CERA in the serum of healthy subjects (Locatelli et al.) as well as of NSCLC stage IIIB-IV patients (Hirsh et al). As CERA, which is pegylated, is not cleared by the kidney, it was hypothesized that the clearance of CERA in the blood stream is only accomplished by binding to EpoR and internalization, as seen in *the in vitro* experiments. Furthermore, it was assumed that the main difference between healthy subjects and NSCLC patients in Epo dynamics is the number of CFU-E cells, which may be reduced by the tumor load and by the chemotherapy. Indeed, these assumptions were sufficient to describe the experimental PK data for both healthy subjects and cancer patients (Fig. 5a). Furthermore, the model determined a decrease of 72% in the average number of CFU-E cells in the NSCLC stage IIIB-IV patients, resulting in longer clearance times of CERA.

Then, the inventors applied the same approach to PK data of individual NSCLC patients. While the data appears very heterogeneous, the model could again describe all data sets based only on different numbers of ESA binding sites, i.e. CFU-E cells. While ESA binding sites may also be present on other cells, such as the NSCLC cells, they will not contribute significantly to clearance of CERA due to their low expression levels. Importantly, it was possible to determine the number of CFU-E cells for each cancer patient, showing a high patient-to-patient variability (Fig. 5c).

**Determination of the number of CFU-E cells in healthy subjects and NSCLC patients based on the patient Hemoglobin (Hb) levels.**

The above model was also able to correlate the hemoglobin (Hb) increments with the PK/PD data in individualized patient data sets. The PK profiles correlates with the number of CFU-E and this number with the recovery of the anemia, indicated by Hb levels. The inventors established the correlation between the individual patient histories with the PK profiles and these ones with the number of CFU-E per patients, and these ones with the outcome of the ESA treatment (increment of Hb levels). The Hb model includes therefore the additional reactions (figure 6c) of the production of Hb by active ESA-EPO-R signalling since the ESA-EPO-R signalling induces the maturation of erythrocytes that therefore increases Hb concentrations. Additionally, the model includes the patient specific degradation of Hb, which is easily determined in anemic patients, because there Hb status is regularly monitored.

**Example 1: Model-based patient stratification**

An increase in risk of mortality has been associated with ESA treatment in cancer-associated anaemia. The inventors used logistic regression to correlate the two inferred patient-specific parameters (number of ESA binding sites and Hb degradation rate) and the given ESA treatment with fatal outcome (Fig. 7a) in two NSCLC clinical trials (NCT00072059 and NCT00327535). The Youden's index was used to define the prognostic value of the two patient-specific parameters and the given dose of C.E.R.A., which are visualized individually or in combination (Fig. 7b) with correlation to fatal outcome, defining a threshold for patient stratification. Based on the combination of the number of ESA binding sites, Hb degradation rate and the dose of C.E.R.A. given in the first three weeks, the model was able to classify the patients into high or low risk of fatal outcome, and showed a good correlation between patients in the high risk group and the deceased patients (Fig. 8a and Fig. 7c). These results substantiated previous observations that overall survival can be retrospectively correlated with an Hb decrease during chemotherapy or an increase of haemoglobin levels above 13 g/dl or with high ESA dose exposures. Subsequently, the inventors validated the inventor's approach using only PD values and the administered ESA regimens in an independent group of patients in the NSCLC clinical trial (NCT00072059). The mathematical model was capable to correctly assign the relative majority of the deceased patients to the high risk group of fatal outcome (Fig. 8b).

Previous attempts to statistically identify risk factors for mortality and thrombovascular events upon ESA treatment pointed to high ESA doses, hyporesponsiveness to treatment or Hb levels >13 g/dl. However, these risk factors can only be obtained retrospectively. With the inventor's multiscale model it is possible to estimate the two patient-specific parameters for each individual patient already after a few measurements of Hb. Based on these parameters the multiscale model is capable to predict the minimal effective dose and to stratify patients to low or high risk of mortality. The inventor's approach also provides a pharmacovigilance tool to retrospectively asses the risk/benefit in ESA safety studies. The threshold for being at high risk for an adverse event is provided in figure 9.

**Claims**

1. A method for stratifying an anemia patient who receives treatment with an erythropoiesis Stimulating Agents (ESA), wherein the patient is stratified into a high risk or low risk group of experiencing an adverse event upon continued treatment with the ESA, the method comprising the steps of:

   (a) Providing patient samples of the patient from at least two time points during the initial treatment of anemia with the ESA in said patient,
   (b) Determining from said samples the individual hemoglobin (Hb) degradation rate [Hb degr] and number of ESA binding sites [EpoR] for said patient,
   (c) Determining from (i) the individual Hb degradation rate, (ii) the number of ESA binding sites, and (iii) the last ESA dose administered, or ESA dose planned to be administered, to said patient [ESA], an accumulated risk factor [aRF], and
   (d) Stratifying the patient into a high risk or low risk group of experiencing an adverse event upon continued treatment with the ESA according to the [aRF].

2. The method according to claim 1, wherein the [aRF] is determined according to the following equation (1):

$$(1)\ [aRF]=B0+B1*[EpoR]+B2*[Hb\ degr]+B3*[ESA]$$

3. The method according to claim 1 or 2, wherein the ESA is selected from Continuous erythropoietin receptor activator (CERA), EPO alfa, EPO beta, and novel erythropoiesis-stimulating protein (NESP), and preferably is CERA.

4. The method according to claim 2, wherein B0=2.3518, B1=-2.5840, B2=-0.3957, and B3=-0.1374, and preferably wherein the patient is stratified into a high group of experiencing an adverse event upon continued treatment with the ESA if the [aRF] is larger than about 0.18.

5. The method according to any one of claims 1 to 4, wherein the number of ESA binding sites [EpoR] for said patient is determined by

   (a) assessing the clearance of the administered ESA in the serum of said patient over time, and
   (b) Calculating from the clearance of said ESA using a non-linear dynamic pharmacokinetic (PK) ESA-EPO-R pathway model the amount of ESA binding sites in said patient [EpoR].

6. The method according to any one of claims 1 to 5, wherein the individual hemoglobin (Hb) degradation rate [Hb degr] is determined by calculating from the hemoglobin concentration of the patient from at least two separate time points the patient's individual hemoglobin degradation rate (degradation of hemoglobin per time).

7. The method according to any one of claims 1 to 6, wherein the patient samples are blood samples.

8. The method according to claim 5, wherein said non-linear dynamic pharmacokinetic (PK) ESA-EPO-R pathway model is based on a system of the ordinary differential equations (ODE):

$$(2.1.)\qquad \frac{d[ESA_{SC}]}{dt} = -k_{sc_{clear}} \cdot [ESA_{SC}]/(k_{sc\_clear\_sat} + [ESA_{SC}]) - k_{sc\_out}$$

$$[ESA_{SC}]$$

$$(2.2.) \qquad \frac{\mathrm{d}[ESA]}{\mathrm{d}t} = k_{sc_{out}} \cdot [ESA_{SC}] - k_{clear} \cdot [ESA] - k_{on} \cdot [ESA] \cdot [EpoR] + k_{off} \cdot [ESAEpoR] + k_{ex} \cdot [ESAEpoR_i]$$

$$(2.3.) \qquad \frac{\mathrm{d}[EpoR]}{\mathrm{d}t} = -k_{on} \cdot [ESA] \cdot [EpoR] + k_{off} \cdot [ESAEpoR] + k_t \cdot B_{max} - k_t \cdot [EpoR] + k_{ex} \cdot [ESAEpoR_i]$$

$$(2.4.) \qquad \frac{\mathrm{d}[ESAEpoR]}{\mathrm{d}t} = k_{on} \cdot [ESA] \cdot [EpoR] - k_{off} \cdot [ESAEpoR] - k_e \cdot [ESAEpoR]$$

$$(2.5.) \qquad \frac{\mathrm{d}[ESAEpoR_i]}{\mathrm{d}t} = k_e \cdot [ESAEpoR] - k_{ex} \cdot [ESAEpoR_i] - k_{di} \cdot [ESAEpoR_i] - k_{de} \cdot [ESAEpoR_i]$$

$$(2.6.) \qquad \frac{\mathrm{d}[dESA_i]}{\mathrm{d}t} = k_{di} \cdot [ESAEpoR_i]$$

$$(2.7.) \qquad \frac{\mathrm{d}[dESA_e]}{\mathrm{d}t} = k_{de} \cdot [ESAEpoR_i],$$

and
wherein $B_{max}$ is the number of ESA binding sites.

9. The method according to any one of claims 1 to 8, wherein the anemia is an anemia associated with a cancer disease, chemotherapy induced anemia, or anemia associated with chronic inflammation.

10. An ESA for use in the treatment of anemia in a patient, wherein the patient has a low risk of an adverse event upon continued treatment with the ESA as determined with a method according to any one of claims 1 to 9.

11. The ESA for use according to claim 10, wherein the ESA is selected from Continuous erythropoietin receptor activator (CERA), EPO alfa, EPO beta, and novel erythropoiesis-stimulating protein (NESP), and preferably is CERA.

12. The ESA for use according to claim 10 or 11, wherein the patient is suffering from anemia associated with a cancer disease, chemotherapy induced anemia, or anemia associated with chronic inflammation.

13. The ESA for use according to any one of claims 10 to 12, wherein the treatment comprises the obtaining blood samples of said patient in the first 1 to 5 weeks of the ESA treatment, and calculating therefrom the patient's individual risk of an adverse eventu-pon continued treatment with the ESA by a method according to any one of claims 1 to 9.

14. The ESA for use according to any one of claims 10 to 13, wherein the patient is suffering from anemia as a secondary pathology induced by another disorder such as chronic inflammation, myelodysplastic syndrome or cancer, preferably lung cancer.

15. The ESA for use according to any of claims 10 to 13, wherein the treatment comprising the steps of

(a) Administering to the patient a low ESA dose for the first 1 to 5 weeks, preferably 3 weeks,

(b) Obtaining at least two samples from the patient during the first 1 to 5 weeks, preferably 3 weeks,

(c) Determining from said at least two samples the patient's risk of an adverse event upon continued treatment with the ESA,

(d) Administering to the patient ESA after the first 1 to 5 weeks if the patient is at low risk of an adverse event upon continued treatment with the ESA, or

(e) Administering to the patient a blood transfusion after the first 1 to 5 weeks if the patient is at high risk of an adverse event upon continued treatment with the ESA.

Figure 1:

Figure 1 continued:

Figure 1 continued:

**C**

EpoR affinities for different ESAs

Figure 2:

Figure 2 continued:

Figure 3:

Figure 4:

**A**

Model prediction:
Dose-response in H838-EpoR

Experimental validation
Dose-response in H838-EpoR

**B**

Time-course in H838-EpoR

Time-course in H838

Figure 5:

Figure 6:

**a)  *in vitro* trafficking model**

**b)  *in vivo* PK/PD model**

**C)  *in vivo* PK/PD model**

Figure 7:

Figure 7 cont.:

**b** Predictors for risk model

ROC curve

Combination of
the three predictors

**O** Maximum value of
Youden's index

**c** Risk model calibration based on PK and PD data

Figure 8:

a  Risk model calibration based on PK and PD data (N=205)   b  Risk model validation based on PD data (N=113)

Figure 9:

Europäisches
Patentamt

European
Patent Office

Office européen
des brevets

# EUROPEAN SEARCH REPORT

**Application Number**

EP 17 15 3796

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/020043 A1 (HOFFMANN LA ROCHE [CH]; ROCHE DIAGNOSTICS GMBH [DE]; AMANN ZALAN ILDIK) 21 February 2008 (2008-02-21) | 10-15 | INV.<br>G01N33/72 |
| A | * the whole document * | 1-9 | |
| X | KATODRITOU E ET AL: "Update on the use of erythropoiesis-stimulating agents (ESAs) for the management of anemia of multiple myeloma and lymphoma", CANCER TREATMENT REVIEWS, ELSEVIER, AMSTERDAM, NL, vol. 35, no. 8, 1 December 2009 (2009-12-01), pages 738-743, XP026774946, ISSN: 0305-7372, DOI: 10.1016/J.CTRV.2009.08.002 [retrieved on 2009-09-03] | 10-15 | |
| A | * the whole document * | 1-9 | |
| X | WO 2009/089039 A1 (CHO DANIEL J [US]) 16 July 2009 (2009-07-16) | 10-15 | TECHNICAL FIELDS SEARCHED (IPC) |
| A | * the whole document * | 1-9 | G01N |
| X | EP 2 957 293 A1 (DEUTSCHES KREBSFORSCH [DE]) 23 December 2015 (2015-12-23) | 10-15 | |
| A | * the whole document * | 1-9 | |

The present search report has been drawn up for all claims

| Place of search | Date of completion of the search | Examiner |
|---|---|---|
| Munich | 19 May 2017 | Moreno de Vega, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 15 3796

This annex lists the patent family members relating to the patent documents cited in the above-mentioned European search report.
The members are as contained in the European Patent Office EDP file on
The European Patent Office is in no way liable for these particulars which are merely given for the purpose of information.

19-05-2017

| Patent document cited in search report | | | Publication date | Patent family member(s) | | | Publication date |
|---|---|---|---|---|---|---|---|
| WO 2008020043 | A1 | | 21-02-2008 | AT | 544072 | T | 15-02-2012 |
| | | | | AU | 2007285733 | A1 | 21-02-2008 |
| | | | | BR | PI0716614 | A2 | 08-10-2013 |
| | | | | CA | 2659423 | A1 | 21-02-2008 |
| | | | | CN | 101506660 | A | 12-08-2009 |
| | | | | EP | 2052256 | A1 | 29-04-2009 |
| | | | | ES | 2380498 | T3 | 14-05-2012 |
| | | | | JP | 5084833 | B2 | 28-11-2012 |
| | | | | JP | 2010501073 | A | 14-01-2010 |
| | | | | KR | 20090034980 | A | 08-04-2009 |
| | | | | US | 2008050749 | A1 | 28-02-2008 |
| | | | | WO | 2008020043 | A1 | 21-02-2008 |
| WO 2009089039 | A1 | | 16-07-2009 | US | 2010261648 | A1 | 14-10-2010 |
| | | | | WO | 2009089039 | A1 | 16-07-2009 |
| EP 2957293 | A1 | | 23-12-2015 | EP | 2957293 | A1 | 23-12-2015 |
| | | | | EP | 3157545 | A2 | 26-04-2017 |
| | | | | US | 2017128532 | A1 | 11-05-2017 |
| | | | | WO | 2015193462 | A2 | 23-12-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015193462 A

### Non-patent literature cited in the description

- **WEISS ; GOODNOUGH.** *N.Engl. J Med,* 2005
- **BECKER V et al.** *Science,* 11 June 2010, vol. 328 (5984), 1404-8
- **RAUE et al.** *PloS ONE,* 2013
- **KREUTZ et al.** *Bioinformatics,* 2007
- **RAUE et al.** *Bioinformatics,* 2011
- **YOUDEN, W. J.** Index for rating diagnostic tests. *Cancer,* 1950, vol. 3, 32-35